# EUROPEAN PATENT SPECIFICATION

(11) **EP 3 193 762 B1**
(45) Date of publication and mention of the grant of the patent: **03.04.2024**
(21) Application number: 15846240.8
(22) Date of filing: 17.09.2015
(51) Int. Cl.: A61B 18/14, A61B 18/00

(54) **ELECTROSURGERY PROBES WITH SMOKE AND LIQUID EVACUATION**
ELEKTROCHIRURGISCHE SONDEN MIT RAUCH- UND FLÜSSIGKEITSABFÜHRUNG
SONDES D'ÉLECTROCHIRURGIE À ÉVACUATION DE FUMÉE ET DE LIQUIDE

(30) Priority: 17.09.2014 US 201414489292
(43) Date of publication of application: 26.07.2017
(73) Proprietor: Zinnanti, William J., Santa Cruz, California 95062 (US)
(72) Inventor: Zinnanti, William J., Santa Cruz, California 95062 (US)
(74) Representative: Gill, David Alan
(86) International application number: PCT/US2015/050674
(87) International publication number: WO 2016/053635

(56) References cited:
- EP-A1- 2 550 924
- US-A1- 2004 024 397
- US-A1- 2004 230 190
- US-A1- 2007 106 292
- US-A1- 2010 205 802
- US-A1- 2011 288 392
- US-A1- 2012 035 605
- US-A1- 2013 012 934
- US-A1- 2014 046 321
- US-B2- 7 329 253

## Description

### TECHNICAL FIELD

This invention relates generally to surgical instruments. More particularly, the invention relates to electro surgery probes and electro surgery probe systems that provide removal of smoke, liquids, and debris during surgical operations.

### BACKGROUND OF THE INVENTION

In certain deep surgical procedures, it is necessary to operate through a small opening into the body. These surgeries commonly employ specula (medical instruments for dilating a bodily passage or cavity) with built-in tubes to remove smoke from the surgical site during procedures such as laser or electrosurgical excision or cauterization. Additionally, a separate suction tube and/or irrigation source may be introduced for the purpose of removing smoke and debris from, or irrigation of, the surgical site. Tubing used to remove smoke and debris or to provide irrigation can obstruct the surgical site and impede visualization. Therefore, it is preferable to avoid having these additional tubes at the surgical site.

Systems used in electro surgery procedures (also referred to herein as electrosurgical procedures) typically comprise a handpiece and interchangeable electrodes or probes. Electro surgery probes consist of a tip that contacts tissue at the surgical site, a shaft of a given length, and a connector end that is inserted into the handpiece. The tips are made in many different shapes and sizes, including blade, ball tip, needle tip, and thin wire loops.

Prior art electro surgery systems that provide evacuation and/or irrigation have a variety of disadvantages. For example, electro surgery devices with incorporated suction were previously described in U.S. Patent No. 2,888,928 to Seiger, U.S. Patent No. 3,828,780 to Morrison, and U.S. Patent No. 4,562,838 to Walker. These devices comprise a hollow metal conductive tube covered by insulation. The hollow metal tube carries the suction and ends in a blunt tip. Smoke enters the metal tube through the blunt tip that is formed by one end of the tube and is evacuated through the other end of the metal tube, flowing through the tube from one end to the other. This design is limited to a single tip that can perform coagulation but not cutting and requires a disposable handpiece with a smoke evacuation channel running through the handpiece.

U.S. Patent No. 5,234,428 to Kaufman, U.S. Patent No. 5,242,442 to Hirschfeld, and U.S. Patent No. 8,057,470 to Lee et al. describe devices that include a smoke suction tube that surrounds or is applied next to a standard detachable electrode or blade. These devices also require a disposable handpiece with a smoke evacuation channel running through the handpiece.

Other patents for electro surgery systems with incorporated smoke evacuation include U.S. Patent No. 5,836,944 to Cosmescu, U.S. Patent No. 5,224,944 to Elliott, and U.S. Patent No. 6,146,353 to Piatt. These patents describe a moveable or detachable shroud or tube that covers or lies next to the electro surgery probe and provides the smoke evacuation conduit. Exchanging of electrodes or probes to provide different tips requires removal and/or adjustment and readjustment of the smoke evacuation conduit.

US Patent Application Publication No. 2004/0230190 A1 relates to systems, apparatus, and methods for ablating, sculpting, severing, shrinking, coagulating, or otherwise modifying a target tissue to be treated.

Therefore, it would be desirable to provide an electrosurgery probe and electro surgery system that overcome the aforementioned and other disadvantages.

### SUMMARY OF THE INVENTION

The invention is set out in the apendend set of claims.

The advantages of the invention will become further apparent from the following detailed description of the presently preferred embodiments, read in conjunction with the accompanying drawings, which are not to scale. The detailed description and drawings are merely illustrative of the invention, rather than limiting, the scope of the invention being defined by the appended claims.

### BRIEF DESCRIPTION OF THE DRAWINGS

FIG. 1A is a perspective view of an electro surgery system;
**FIG. 1B** is an exploded view of the system of **FIG. 1A****;**
**FIG. 1C** is an enlarged view of a portion of the electrosurgery probe illustrated in **FIGS. 1A** and **1B****;**
**FIG. 2A** is a perspective view of an electrosurgery probe, the electrosurgery probe having a thin wire loop tip;
**FIG. 2B** is a perspective view of an electrosurgery probe, the electrosurgery probe having a ball tip;
**FIG. 2C** is a perspective view of an electrosurgery probe, the electrosurgery probe having a needle tip;
**FIG. 2D** is a perspective view of an electrosurgery probe, the electrosurgery probe having a blade tip;
**FIG. 2E** is a perspective view of an electrosurgery probe, the electrosurgery probe shown curved;
**FIG. 2F** is a perspective view of an electrosurgery probe, the electrosurgery probe shown angled;
**FIGS. 3A-3C** provide cross-sectional views of a shaft of an electrosurgery probe;
**FIG. 4A** is a perspective view of an electrosurgery probe or electrosurgery probe conductive element in accordance with the present invention, the electrosurgery probe or electrosurgery probe conductive element having a blade tip;
**FIG. 4B** is a perspective view of an electrosurgery probe or electrosurgery probe conductive element in accordance with the present invention, the electrosurgery probe or electrosurgery probe conductive element having a blunt tip;
**FIG. 4C** is a perspective view of an electrosurgery probe or electrosurgery probe conductive element in accordance with the present invention, the electrosurgery probe or electrosurgery probe conductive element having an elongated mid-segment and a blade tip;
**FIGS. 4D** and **4E** are perspective views of an electrosurgery probe or electrosurgery probe conductive element in accordance with the present invention, the electrosurgery probe or electrosurgery probe conductive element having a flexible mid-segment;
**FIG. 5A** is a perspective view of another electrosurgery system in accordance with the present invention;
**FIG. 5B** is a cutaway view of the handpiece of the system of **FIG. 5A****;**
**FIG. 5C** is a cutaway view of a distal portion of the electrosurgery system of **FIG. 5A** enlarged to show direction of flow during evacuation of smoke and/or liquids or debris; and
**FIG. 6** is a side view of an alternative handpiece.

### DETAILED DESCRIPTION OF THE

### PRESENTLY PREFERRED EMBODIMENTS

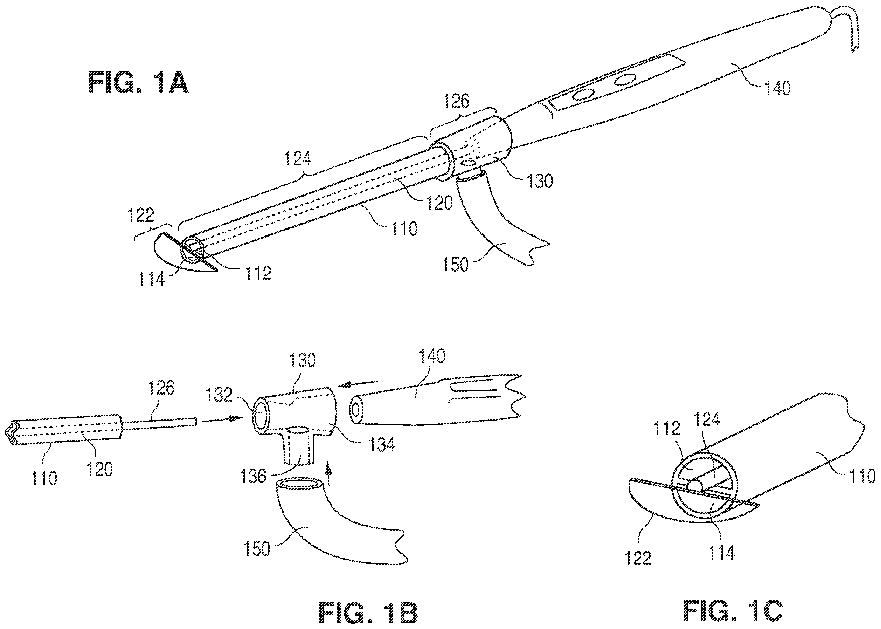

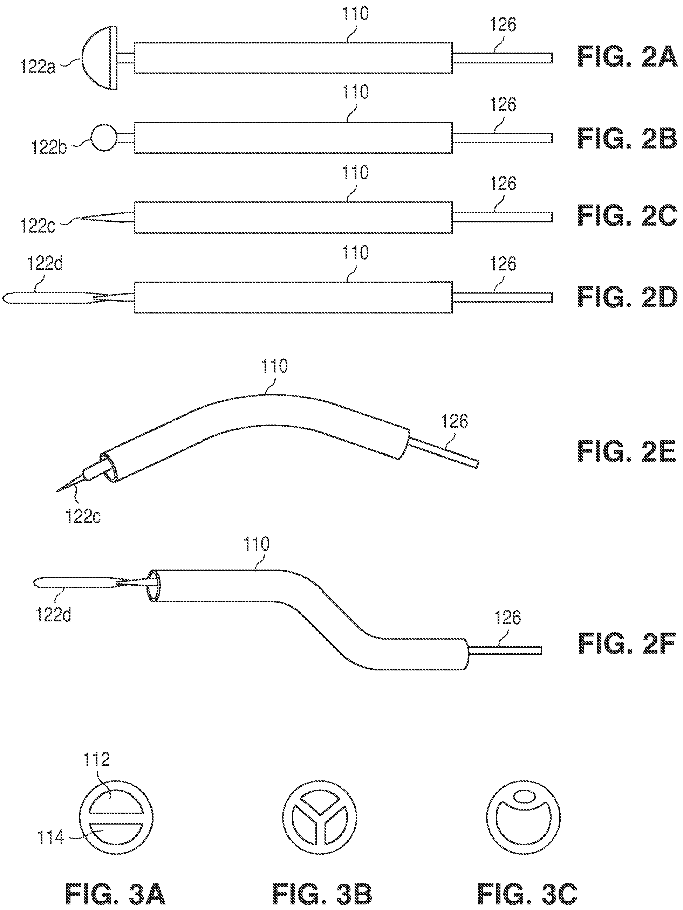

One aspect of the present invention is an electrosurgery probe. Electrosurgery probes in accordance with one embodiment of the present invention are illustrated in **FIGS. 1A** and **1B** as elements of an electrosurgery system and in **FIGS. 2A-2F** independent of the system. Each electrosurgery probe of the present embodiment comprises an insulated shaft **110** and a conductive element **120.**

As illustrated in **FIGS. 1A, 1B****,** and **2A****-2F,** shaft **110** is an elongated, substantially cylindrical member. The length of shaft **110** is variable depending on the intended use of the electrosurgery probe; the shaft must extend out from a connector **130** just far enough to allow space for flow of suction or irrigation when the electrosurgery probe is properly seated in connector **130** and an electrosurgery handpiece **140,** as described below. The shaft need not be cylindrical, with virtually any elongated shape being acceptable. Because the conductive element disposed within shaft **110** is electrically conductive, the shaft is made of an insulating (e.g., dielectric or nonconductive) material having sufficient rigidity to allow the tip segment of the electrosurgery probe to be applied to tissue appropriately during a surgical procedure. The insulating shaft shields from inadvertent lateral burning of tissue along the sides of the electrosurgery probe. This is especially important with elongated probes for deep procedures in body cavities. The insulating material is typically molded plastic or heat-shrink tubing. Suitable materials include elastomers and polymers such as polydimethylsiloxane (PDMS), polymethylmethacrylate (PMMA), polycarbonate, polytetrafluoroethylene (PTFE), polyvinylchloride (PVC), polysulfone, polystyrene, polymethylpentene, polypropylene, polyethylene, polyvinylidine fluoride, ABS (acrylonitrile-butadiene-styrene copolymer), cyclic-olefin polymer (COP), cyclic-olefin copolymer (COC), and other insulating materials that prevent or minimize conduction of both heat and electricity. The shaft may be, for example, an extruded or molded plastic tubing having multiple channels formed during the extrusion or molding process.

Shaft **110** includes at least a first channel **112** and a second channel **114.** First channel **112** is substantially occupied by conductive element **120.** Second channel **114** is open the entire length of the shaft and available for suction and/or irrigation supplied, for example, by a length of tubing **150** with which channel **114** is in fluid communication. Channels **112** and **114** are covered channels (i.e., lumens) that extend the entire length of shaft **110.** **FIGS. 3A-3C** show various possible cross-sectional views of shaft **110,** with channels **112** and **114** indicated in **FIG. 3A****.** One skilled in the art will appreciate that **FIGS. 3A-3C** represent just a few of many possible cross-sectional configurations.

Conductive element **120** comprises a tip segment **122,** a mid-segment **124,** and an electrical contact segment **126,** all of which can be seen in **FIG. 1A****.** Tip segment **122** may assume a variety of different shapes such as, for example, a thin wire loop (**FIGS**. **1A** and **1C** at **122** and **FIG. 2A** at **122a**) or blade tip (**FIGS**. **2D** and **2F** at **122d**) for cutting, a ball tip (FIG. 2B at **122b**) for coagulation after cutting, or a needle tip (**FIGS**. **2C** and **2E** at **122c**) for transfer of concentrated energy into a limited area. Other shapes are, of course, possible. As is evident from **FIGS. 2A-2F****,** each electrosurgery probe according to the current invention has a single tip shape. The entire electrosurgery probe can be exchanged for another electrosurgery probe during a surgical procedure if a different tip shape is desired. Mid-segment **124** and electrical contact segment **126** are typically a stainless steel rod or wire but may, alternatively, be constructed using any conductive material capable of enabling the probe to perform an electrosurgery procedure. Tip segment **122** may be constructed of the same material as the mid-segment and electrical contact segment; loop tips are also commonly fabricated from tungsten wire.

As seen in **FIG. 1A****,** conductive element **120** is disposed in channel **112** such that tip segment **122** extends from a distal end of shaft **110,** mid-segment **124** is within shaft **110,** and electrical contact segment **126** extends from a proximal end of shaft **110.** (The term "distal" is used herein to designate an end or portion nearest to the patient during use of the electrosurgery probe, and the term "proximal" is used herein to designate an end or portion nearest to the operator during use of the electrosurgery probe.) During fabrication of the electrosurgery probe, conductive element **120** may be positioned in shaft **110** by being inserted into a fully formed shaft. Alternatively, where shaft **110** is an extruded or molded plastic tubing having multiple channels formed during the extrusion or molding process, the tubing may be extruded or molded directly over conductive element 120. Extrusion or molding of the shaft over the conductive element is particularly convenient where the mid-segment of the electro surgery probe is curved or angled as seen in FIGS. 2E and 2F, respectively.

Another aspect of the present invention is an electro surgery system. In one embodiment, the system is formed when a connector 130 serves as a common hub to bring together an electro surgery probe such as has been described above, an electro surgery handpiece 140, and a length of tubing 150. In the system, both the electro surgery probe and handpiece 140 are removably attached to connector 130. The system may include multiple interchangeable electro surgery probes to offer a variety of different electro surgery tips such as can be seen in FIGS. 2A-2F.

As illustrated in FIGS. 1A and IB, connector 130 is a T-connector having first, second, and third connection points 132, 134, and 136, respectively. While connector 130 is shown to have three connection points, the connector may, alternatively, have more than three connection points and may assume shapes other than that of the T- connector shown in FIGS. 1A and IB. For example, connector 130 could be a Y-connector. Connector 130 is preferably made of a soft rubber or plastic material but can be made of other materials that are conformable to one or both of the shaft 110 of the electro surgery probe and the handpiece 140. Connector 130 is of sufficient size to adapt to different sizes and shapes of standard electro surgery handpieces.

As indicated in FIGS. 1A and IB, a proximal portion of the electro surgery probe is removably disposed within connector 130 at connection point 132 such that electrical contact segment 126 of the electro surgery probe is fully within connector 130 and a substantially air-tight connection is made between shaft 110 and connector 130. Connection point 132 is illustrated in FIGS. 1A and IB as a female connection point; however, it will be appreciated that shaft 110 could be constructed such that a portion of shaft 110 fits over a male connection point 132 rather than within a female connection point 132.

A distal portion of electro surgery handpiece 140 is removably disposed within connection point 134 such that a substantially air-tight connection is made between the electro surgery handpiece and the connector and such that electrical contact segment 126 of the electro surgery probe is inserted into electro surgery handpiece 140, thereby providing an electrical connection between the electrosurgery probe and electro surgery handpiece 140. The electrosurgery handpiece may be any commercially available handpiece and may be, for example, either hand activated or foot activated. Thus, it is preferable that connection point 134 be a female connection point that can be slipped over the distal end of the desired handpiece. As mentioned previously, connector 130 may be constructed of a soft rubber or plastic, making the connector readily adaptable to various shapes and sizes of handpieces.

One end of a length of tubing 150 is either removably or permanently attached to connector 130 at connection point 136, which may be either a male (as illustrated) or female connection point. The other end of tubing 150 is attached to, for example, a vacuum source or an irrigation fluid source. Connector 130, the electrosurgery probe, and/or tubing 150 may be adapted such that both a vacuum source and an irrigation fluid source may be simultaneously connected to the electrosurgery probe via connector 130. Thus, channel 114 of the electrosurgery probe may be in fluid communication with a vacuum and/or irrigation fluid source via tubing 150 and connector 130.

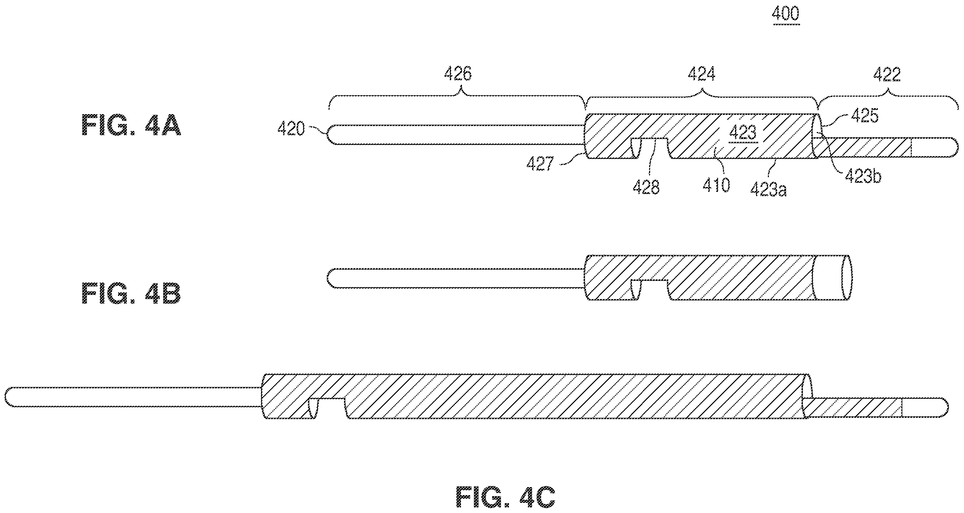

A conductive element for an electro surgery probe according to the invention can be seen at 420 in FIGS. 4A-4E. Conductive element 420 comprises a tip segment 422, a mid-segment 424, and an electrical contact segment 426.

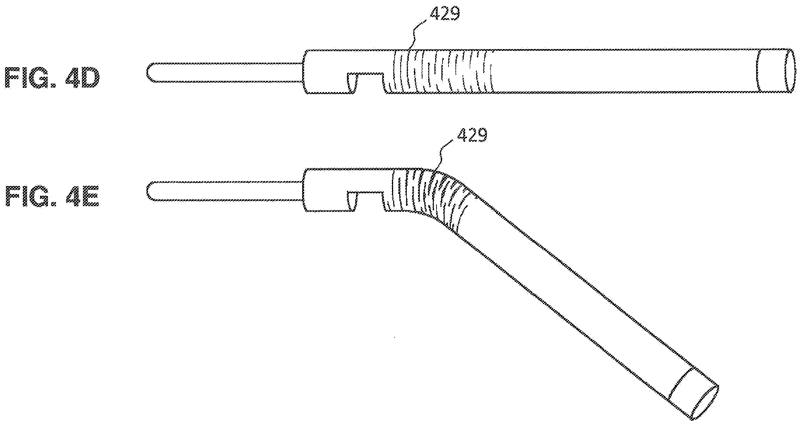

In the present embodiment, both tip segment 422 and mid-segment 424 are segments of a hollow tube 423 (i.e., a tube having a wall 423a and an interior flow passage 423b). Tip segment 422 is formed into hollow tube 423 by, for example, laser cutting the desired shape into the distal end of the tube. Desired shapes may be, for example, a blade tip as seen in FIGS. 1A and 1C or a blunt tip as seen in FIGS. 4B, 4D, and 4E. Other tip shapes are possible. In this embodiment, the wall of tube 423 is constructed using any conductive material capable of performing an electrosurgery procedure. One such conductive material is stainless steel.

Alternatively, the mid-segment may comprise a hollow tube, while the tip segment may be a blade (or other shape, including ball and needle such as can be seen in **FIGS. 2B** and **2C**) that is formed separately and attached to the mid-segment by, for example, welding or any other appropriate attachment process. Where the tip segment is formed separately, the wall of the hollow tube forming the mid-segment need not itself comprise a conductive material if a conductive path is provided between the tip segment and the electrical contact segment by a conductive element such as, for example, a thin strip of metal or a wire extending the length of the mid-segment and connecting to both the electrical contact segment and the tip segment.

The mid-segment may be any desired length as illustrated by **FIGS. 4A** through **4C****,** allowing for use in both superficial and deep procedures. The inner diameter of the hollow tube in the area of the mid-segment is preferably large enough to provide adequate suction for removing blood and other fluids and debris from the procedure site. The distal end **425** of mid-segment **424** is open, while the proximal end **427** is closed. At least one opening **428,** e.g., a notch as seen in **FIGS. 4A-4E****,** is cut or otherwise formed through wall **423a** adjacent to (i.e., at or near) the proximal end of mid-segment **424.** Opening **428** is in fluid communication with interior flow passage **423b** and is preferably at least as large as the opening at the distal end of the conductive element (e.g., at least as large as the cross-sectional area of the interior flow passage) to avoid restriction of flow according to Bernoulli's principle. More than one opening **428** may be formed through the wall of the mid-segment. For example, it may be advantageous to provide a second opening **428** rotated 180° about the external surface of the tube from the location of the first opening **428.**

The mid-segment may include a series of cuts **429** extending through the wall of the tube as illustrated in **FIGS. 4D** and **4E****.** The cuts result in a flexible mid-segment, allowing the tube to be bent into any desired shape without significantly reducing the cross-sectional area of the interior flow passage. Preferably the tube is conductive, e.g., comprises a metal. **FIGS. 4D** and **4E** show cuts within a limited portion of the mid-segment; however, the cuts could extend over any desired portion of the mid-segment, permitting the mid-segment to be shaped not only as seen in **FIG. 4E****,** but also into additional shapes such as, for example, those seen in **FIGS. 2E** and **2F****.** The series of cuts preferably forms an interrupted spiral pattern; i.e., the series of cuts goes around the tube approximately 1/3 of the circumference of the tube and ends and then begins again. The cuts preferably have a .02-inch to.04-inch pitch, the pitch being the nominal distance between two adjacent cuts.

Electrical contact segment **426** may be a separate piece welded or otherwise affixed to the hollow tube forming tip segment **422** and mid-segment **424.** Alternatively, electrical contact segment **426** may be an extension of the hollow tube where the hollow tube is itself conductive. Electrical contact segment **426** comprises a conductive material capable of enabling the probe to perform an electrosurgery procedure. For example, where the probe is used for performing a cautery procedure, the conductive material is capable of heating tip segment **422** to an appropriate temperature for performing the procedure (e.g., between approximately 350 °C and 1200 °C).

The mid-segment may include an insulating (e.g., dielectric or nonconductive) coating **410** disposed over an external surface of the wall of the hollow tube forming the mid-segment. Suitable materials for insulating coating **410** include elastomers and polymers such as polydimethylsiloxane (PDMS), polymethylmethacrylate (PMMA), polycarbonate, polytetrafluoroethylene (PTFE), polyvinylchloride (PVC), polysulfone, polystyrene, polymethylpentene, polypropylene, polyethylene, polyvinylidine fluoride, ABS (acrylonitrile-butadiene-styrene copolymer), cyclic-olefin polymer (COP), cyclic-olefin copolymer (COC), and other insulating materials that prevent or minimize conduction of both heat and electricity. In one example, the insulating coating is disposed over at least a portion of an external surface of mid-segment **424** and may be, for example, a heat-shrink plastic. A polyolefin insulation may also be applied as well as high-temperature Teflon^{®} (polytetrafluoroethylene [PTFE]) for extra heat resistance.

Insulating coating **410** plus conductive element **420** together may form an electrosurgery probe **400** as indicated in **FIG. 4A****.** Note that an insulating coating may not be a required element of electrosurgery probe **400,** for example when the wall of the hollow tube forming the mid-segment is itself non-conductive. Where an insulating coating is not necessary, the conductive element alone serves as an electrosurgery probe. Coated or uncoated conductive elements having blade or needle tips typically serve as dissecting electrosurgery probes. Coated or uncoated conductive elements having blunt tips typically serve as suction/coagulator electrosurgery probes.

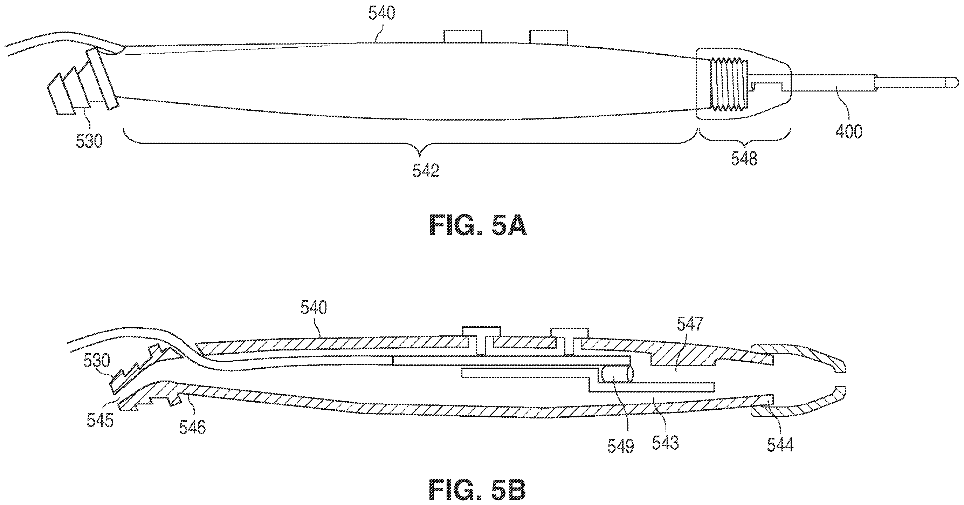

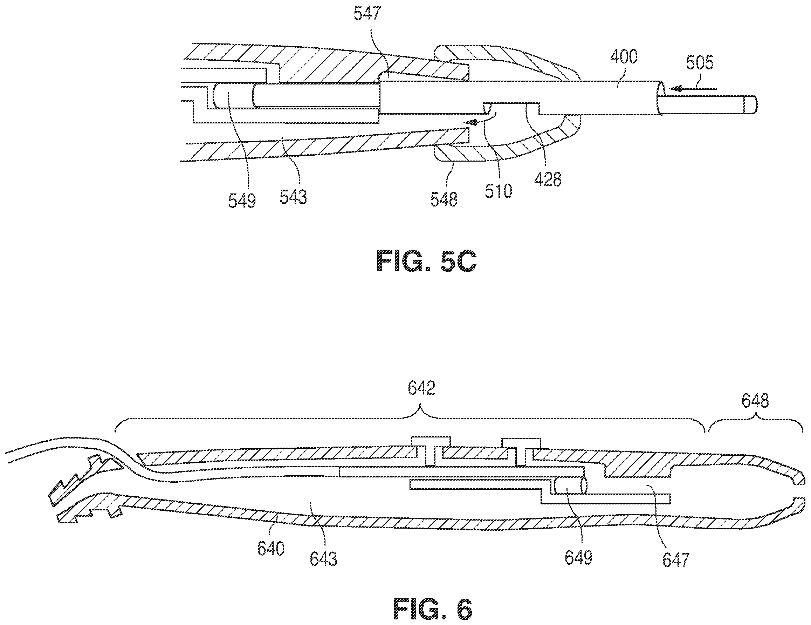

The electrosurgery probes described above and illustrated in **FIGS. 4A-4E** may be combined with an electrosurgery handpiece such as is seen at **540** in **FIGS. 5A-****5C** to form an electrosurgery system. Note that an alternative handpiece is illustrated in **FIG. 6** and is described below. The electrosurgery system may include multiple interchangeable electrosurgery probes to offer a variety of different electrosurgery tips. A first electrosurgery probe may be removed and exchanged for a second electrosurgery probe during a surgical procedure.

Handpiece **540** comprises elongate body segment **542** and exhaust segment **548.** Body segment **542** is typically constructed of a substantially rigid and nonconductive material such as ABS or PVC. A main flow passage **543** extends longitudinally through handpiece **540** from the distal end **544** of body segment **542** to an opening **545** adjacent to (i.e., at or near) the proximal end **546** of body segment **542.** Opening **545** is in fluid communication with a vacuum source (i.e., a source of suction or negative pressure) via a connector **530.**

A second passage **547** extends longitudinally through a distal portion of body segment **542** and terminates in an electrical contact **549.** As illustrated, electrical contact **549** is a concentric electrical receptacle comprising a metal tube, for example a copper tube, that is split along at least a portion of its length, allowing the tube to expand sufficiently to receive the electrical contact segment of the desired electrosurgery probe. The copper tube is connected to a power source (not shown) when the electrosurgery system is in operation. Thus the copper tube provides a conductive path from the power source to electrical contact segment **426** via the copper tube and then on through mid-segment **424** to tip segment **422.** Alternatively, electrical contact **549** may be any appropriate electrical contact known in the art, one such being simply an exposed end of an electric power cable.

When the electrosurgery system is in use, the electrical contact segment **426** of an electrosurgery probe **400** is removably disposed within handpiece **540** such that at least a portion of the electrical contact segment is within second passage **547** and in contact with electrical contact **549,** thereby providing an electrical connection between electrosurgery handpiece **540** and electrosurgery probe **400.** Second passage **547** may be tubular or may be a series of aligned rings or partial rings through which electrical contact segment **426** is directed to electrical contact **549.** Both handpiece **540** and electrosurgery probe **400** are dimensioned to ensure correct positioning of electrical contact segment **426** within second passage **547.**

As seen in **FIGS. 5A-5C****,** exhaust segment **548** is a cap that is formed separately from body segment **542** and attached either permanently or removably to the distal end **544** of body segment **542** by any appropriate bonding or other attachment method. Exhaust cap **548** is constructed of a material such as PVC or silicone rubber that is capable of removably receiving an electrosurgery probe and forming a substantially air-tight seal between the electrosurgery handpiece and the electrosurgery probe.

Exhaust cap **548** is substantially hollow and has an interior cross-sectional diameter that is greater than a cross-sectional diameter of main flow passage **543.** As is best seen in **FIG. 5C****,** the handpiece and electrosurgery probe are dimensioned to ensure that opening **428** formed in mid-segment **424** is positioned within exhaust cap **548** when the electrosurgery probe is inserted into the handpiece. Positioning of opening **428** within exhaust cap **548** ensures that smoke and liquids can flow freely out of opening **428** and into main flow passage **543,** providing fluid communication between the interior of exhaust cap **548** and main flow passage **543.** Arrows **505** and **510** show the direction of flow through mid-segment **424** and into main flow passage **543.** The material of cap **548** may be opaque, translucent, or transparent as seen in **FIG. 5A****.** A transparent cap allows for easy visualization of clots or tissue that may clog the passage and require clearing. Transparency can also be useful for ensuring an electrosurgery probe is correctly inserted into the electrosurgery handpiece.

Alternative handpiece **640,** illustrated in **FIG. 6****,** comprises an elongate body segment **642** and an exhaust segment **648.** As can be seen in **FIG. 6****,** body segment **642** is similar to body segment **542,** which has been described in detail above. However, in the embodiment illustrated in **FIG. 6****,** exhaust segment **648** is an extension of body segment **642** and is formed simultaneously with and of the same material or materials as body segment **642.** For example, both body segment **642** and exhaust segment **648** may be constructed of a material such as PVC that is capable of removably receiving an electrosurgery probe and forming a substantially air-tight seal between the electrosurgery handpiece and the electrosurgery probe.

As can be seen in **FIG. 6****,** exhaust segment **648** is substantially hollow and has an interior cross-sectional diameter that is greater than a cross-sectional diameter of the main flow passage **643.** Both handpiece **640** and the electrosurgery probe are dimensioned to ensure that opening **428** formed in mid-segment **424** of the electrosurgery probe is positioned within exhaust segment **648** when the electrosurgery probe is inserted into handpiece **640.** Positioning of opening **428** within exhaust segment **648** ensures that smoke and liquids can flow freely out of opening **428** and into main flow passage **643,** providing fluid communication between the interior of exhaust segment **648** and main flow passage **643.**

In the present embodiment, when the electrosurgery system is in use, the electrical contact segment **426** of an electrosurgery probe **400** is removably disposed within handpiece **640** such that at least a portion of the electrical contact segment is within a second passage **647** and in contact with an electrical contact **649,** thereby providing an electrical connection between electrosurgery handpiece **640** and electrosurgery probe **400.** Second passage **647** may be tubular or may be a series of aligned rings or partial rings through which electrical contact segment **426** is directed to electrical contact **649** Both handpiece **640** and electrosurgery probe **400** are dimensioned to ensure correct positioning of electrical contact segment **426** within second passage **647.**

In another embodiment, an electro surgery system may be formed by combining a conductive element such as has been described above and illustrated at 420 in FIGS. 4A-4E with an electro surgery handpiece such as is seen in FIGS. 1A and IB. In the present embodiment, conductive element 420 is inserted into connection point 132 of connector 130. I.e., both shaft 110 and conductive element 120 in FIGS. 1A and IB are replaced by a conductive element 420 where the mid-segment 424 is non-conductive, or by a combination of a non-conductive coating 410 and a conductive element 420 where the mid-segment 424 is conductive. Conductive element 420 is positioned within connector 130 such that tip segment 422 and a distal portion of mid- segment 424 extend from connector 130, and electrical contact segment 426 extends through connector 134 into electro surgery handpiece 140. Opening 428 is positioned within connector 130 in fluid communication with connection point 136 and tubing 150. Preferably mid-segment 424 forms a substantially air-tight connection with connector 130 at connection point 132. In this embodiment, simultaneous smoke and liquid evacuation is possible.

An example method of performing an electro surgery procedure, not under the scope of the present invention, includes attaching to a connector, in any order, a first electro surgery probe, an electro surgery handpiece having an activator, and a first length of tubing that is in fluid communication with one or both of a vacuum source and an irrigation fluid source. The electro surgery handpiece is connected to an electrical source either before or after the handpiece is attached to the connector. The resulting electro surgery system is then activated by operating the activator of the handpiece (for example, either a hand or foot activator), and the system is used to perform an electro surgery procedure. The method may further comprise removing the first electro surgery probe from the connector and attaching a second electro surgery probe to the connector. This step may be performed without removing the electro surgery handpiece from the connector and also without removing the attached tube(s) from the connector. The method may also further comprise attaching a second length of tubing to the connector if both vacuum and irrigation are desired, the second length of tubing in fluid communication with one or both of a vacuum source and an irrigation fluid source.

In another example method of performing an electro surgery procedure, not under the scope of the present invention, an electro surgery system is provided that includes an electro surgery handpiece, a dissecting electro surgery probe, and a suction/coagulator electrosurgery probe. The dissecting electro surgery probe is attached to the handpiece. Dissection is performed at an electrosurgery site using the dissecting electrosurgery probe. The dissecting electrosurgery probe is detached from the handpiece, and the suction/coagulator electrosurgery probe is attached to the handpiece. One or both of suction and coagulation is performed at the electrosurgery site.

## Claims

1. An electrosurgery probe for an electrosurgery handpiece (140), comprising:
an electrically-conductive element (400) comprising a surgical tool tip segment (422), a mid-segment (424), and an electrical contact segment (426), the mid-segment comprising a hollow tube (423) having a wall (423a) and an interior flow passage (423b), the mid-segment having an open distal end (425) and a closed proximal end (427), wherein the mid-segment is coupled at its distal end directly to the surgical tool tip segment and coupled at its closed proximal end directly to the electrical contact segment, the mid-segment further having an opening formed through the wall of the tube adjacent to the proximal end of the mid-segment, the opening in fluid communication with the interior flow passage, the tip segment and electrical contact segment extending contiguously from opposite ends of the mid-segment, and wherein
the surgical tool tip segment, the mid-segment and the electrical contact segment are all comprised of at least one electrically-conductive material to enable passage of electrical current from the electrical contact segment through the mid-segment to the surgical tool tip segment.

2. The electrosurgery probe of claim 1 further comprising a coating (410) disposed over at least a portion of an external surface of the mid-segment (424).

3. The electrosurgery probe of claim 2 wherein the coating comprises an insulating material selected from the group consisting of polydimethylsiloxane (PDMS), polymethylmethacrylate (PMMA), polycarbonate, polytetrafluoroethylene (PTFE), polyvinylchloride (PVC), polysulfone, polystyrene, polymethylpentene, polypropylene, polyethylene, polyvinylidine fluoride, ABS (acrylonitrile-butadiene-styrene copolymer), cyclic-olefin polymer (COP), cyclic-olefin copolymer (COC), and combinations thereof.

4. The electrosurgery probe of claim 1 wherein the opening in the mid-segment (424) is at least as large as a cross-sectional area of the interior flow passage; or optionally wherein the tip segment (422) includes one of a blade tip and a blunt tip; or optionally wherein the tip segment (422) is formed into the hollow tube (423); or optionally wherein the tip segment (422) is formed separately from the mid-segment and is attached to the mid-segment.

5. The electrosurgery probe of claim 1 wherein the tube is conductive.

6. The electrosurgery probe of claim 5 wherein the tube includes a series of cuts extending through the wall of the tube, the cuts resulting in a flexible tube; and optionally wherein the series of cuts forms an interrupted spiral pattern.

7. An electrosurgery system, comprising: an electrosurgery handpiece (140) comprising an elongate body segment and an exhaust segment, the handpiece having a main flow passage extending longitudinally therethrough, the handpiece further having a second passage extending longitudinally through a distal portion of the body segment, the second passage terminating in an electrical contact, the exhaust segment having a cross-sectional diameter greater than a cross-sectional diameter of the main flow passage; and an electrosurgery probe according to claim 1, configured to be removably attachable to the electrosurgery handpiece (140) and
a connector (130) couplable to the electrosurgery handpiece (140) and the electrosurgery probe; wherein the electrical contact segment and a portion of the mid-segment are removably disposed within the electrosurgery handpiece such that the electrical contact segment is positioned within the second passage and the mid-segment opening is positioned within the exhaust segment.

8. The electrosurgery system of claim 7 further comprising a coating disposed over at least a portion of an external surface of the mid-segment (424).

9. The electrosurgery system of claim 7 wherein the exhaust segment is a cap that is formed separately from the body segment and attached to a distal end of the body segment, and is constructed of a material that is capable of removably receiving an electro surgery probe.

10. The electrosurgery system of claim 7 wherein the exhaust segment is an extension of the body segment and is formed simultaneously with the body segment, of the same material or materials as the body segment.

11. The electrosurgery system of claim 7 wherein the electrical contact is a concentric electrical receptacle.

12. The electrosurgery system of claim 7 wherein the electrosurgery probe tip segment (422) is formed into the hollow tube.

## Patentansprüche

1. Elektrochirurgische Sonde für ein elektrochirurgisches Handstück (140), die Folgendes umfasst:
ein elektrisch leitfähiges Element (400), das ein Spitzensegment (422) des chirurgischen Instruments, ein mittleres Segment (424) und ein elektrisches Kontaktsegment (426) umfasst, wobei das mittlere Segment ein hohles Rohr (423) umfasst, das eine Wand (423a) und einen inneren Strömungsdurchgang (423b) aufweist, wobei das mittlere Segment ein offenes distales Ende (425) und ein geschlossenes proximales Ende (427) aufweist, wobei das mittlere Segment an seinem distalen Ende direkt mit dem Spitzensegment des chirurgischen Instruments verbunden ist und an seinem geschlossenen proximalen Ende direkt mit dem elektrischen Kontaktsegment verbunden ist, wobei das mittlere Segment weiter eine Öffnung aufweist, die durch die Wand des Rohrs benachbart zu dem proximalen Ende des mittleren Segments ausgebildet ist, wobei die Öffnung in Flüssigkeitsverbindung mit dem inneren Strömungsdurchgang ist, sich das Spitzensegment und elektrische Kontaktsegment fortlaufend von gegenüberliegenden Enden des mittleren Segments erstrecken, und wobei das Spitzensegment des chirurgischen Instruments, das mittlere Segment und das elektrische Kontaktsegment alle aus zumindest einem elektrisch leitfähigen Material bestehen, um einen Durchgang von elektrischem Strom von dem elektrischen Kontaktsegment durch das mittlere Segment zu dem Spitzensegment des chirurgischen Instruments zu ermöglichen.

2. Elektrochirurgische Sonde nach Anspruch 1, die weiter eine Beschichtung (410) umfasst, die zumindest über einem Teil einer äußeren Oberfläche des mittleren Segments (424) angeordnet ist.

3. Elektrochirurgische Sonde nach Anspruch 2, wobei die Beschichtung ein Isoliermaterial umfasst, das aus der Gruppe ausgewählt ist, die aus Folgenden besteht:
Polydimethylsiloxan (PDMS), Polymethylmethacrylat (PMMA), Polycarbonat, Polytetrafluorethylen (PTFE), Polyvinylchlorid (PVC), Polysulfon, Polystyrol, Polymethylpenten, Polypropylen, Polyethylen, Polyvinylidinfluorid, ABS (AcrylnitrilButadien-Styrol-Copolymer), Cyclo-Olefin-Polymer (COP), Cyclo-Olefin-Copolymer (COC) und Kombinationen davon.

4. Elektrochirurgische Sonde nach Anspruch 1, wobei die Öffnung in dem mittleren Segment (424) mindestens so groß ist wie eine Querschnittsfläche des inneren Strömungsdurchgangs; oder optional wobei das Spitzensegment (422) eine einer Klingenspitze und einer stumpfen Spitze einschließt; oder optional wobei das Spitzensegment (422) im hohlen Rohr (423) ausgebildet ist; oder optional wobei das Spitzensegment (422) getrennt von dem mittleren Segment ausgebildet ist und an das mittlere Segment angebracht ist.

5. Elektrochirurgische Sonde nach Anspruch 1, wobei das Rohr leitfähig ist.

6. Elektrochirurgische Sonde nach Anspruch 5, wobei das Rohr eine Reihe von Einschnitten einschließt, die sich durch die Wand des Rohrs erstrecken, wobei die Einschnitte zu einem flexiblen Rohr führen; und optional wobei die Reihe von Einschnitten ein unterbrochenes Spiralmuster bildet.

7. Elektrochirurgisches System, das Folgendes umfasst: ein elektrochirurgisches Handstück (140), das ein längliches Körpersegment und ein Auslasssegment umfasst, wobei das Handstück einen Hauptströmungsdurchgang aufweist, der sich längs dahindurch erstreckt, das Handstück weiter einen zweiten Durchgang aufweist, der sich längs durch einen distalen Teil des Körpersegments erstreckt, wobei der zweite Durchgang in einem elektrischen Kontakt endet, wobei das Auslasssegment einen Querschnittsdurchmesser aufweist, der größer ist als ein Querschnittsdurchmesser des Hauptströmungsdurchgangs; und eine elektrochirurgische Sonde nach Anspruch 1, die zur entfernbaren Anbringung an das elektrochirurgische Handstück (140) und ein Verbindungsstück (130), das mit dem elektrochirurgischen Handstück (140) und der elektrochirurgischen Sonde verbindbar ist, konfiguriert ist; wobei das elektrische Kontaktsegment und ein Teil des mittleren Segments entfernbar innerhalb des elektrochirurgischen Handstücks angeordnet sind, sodass das elektrische Kontaktsegment innerhalb des zweiten Durchgangs positioniert ist und die Öffnung des mittleren Segments innerhalb des Auslasssegments positioniert ist.

8. Elektrochirurgisches System nach Anspruch 7, das weiter eine Beschichtung umfasst, die zumindest über einem Teil einer äußeren Oberfläche des mittleren Segments (424) angeordnet ist.

9. Elektrochirurgisches System nach Anspruch 7, wobei das Auslasssegment eine Kappe ist, die getrennt von dem Körpersegment ausgebildet ist und an einem distalen Ende des Körpersegments angebracht ist, und aus einem Material gefertigt ist, das eine elektrochirurgische Sonde entfernbar aufnehmen kann.

10. Elektrochirurgisches System nach Anspruch 7, wobei das Auslasssegment eine Verlängerung des Körpersegments ist und gleichzeitig mit dem Körpersegment aus dem gleichen Material oder den gleichen Materialien wie das Körpersegment ausgebildet wird.

11. Elektrochirurgisches System nach Anspruch 7, wobei der elektrische Kontakt eine konzentrische elektrische Aufnahmevorrichtung ist.

12. Elektrochirurgisches System nach Anspruch 7, wobei das Spitzensegment (422) der elektrochirurgischen Sonde im hohlen Rohr ausgebildet ist.

## Revendications

1. Sonde d'électrochirurgie destinée à une pièce à main d'électrochirurgie (140), comprenant :
un élément électriquement conducteur (400) comprenant un segment de pointe d'outil chirurgical (422), un segment intermédiaire (424), et un segment de contact électrique (426),
le segment intermédiaire comprenant un tube creux (423) ayant une paroi (423a) et un passage d'écoulement intérieur (423b), le segment intermédiaire ayant une extrémité distale ouverte (425) et une extrémité proximale fermée (427), le segment intermédiaire étant couplé au niveau de son extrémité distale directement au segment de pointe d'outil chirurgical et
couplé au niveau de son extrémité proximale fermée directement au segment de contact électrique, le segment intermédiaire comportant en outre une ouverture formée à travers la paroi du tube adjacente à l'extrémité proximale du segment intermédiaire, l'ouverture étant en communication fluidique avec le passage d'écoulement intérieur, le segment de pointe et
le segment de contact électrique s'étendant de manière contiguë depuis des extrémités opposées du segment intermédiaire, et dans lequel le segment de pointe d'outil chirurgical, le segment intermédiaire et le segment de contact électrique sont tous constitués d'au moins un matériau électriquement conducteur afin de permettre le passage d'un courant électrique depuis le segment de contact électrique à travers le segment intermédiaire jusqu'au segment de pointe d'outil chirurgical.

2. Sonde d'électrochirurgie selon la revendication 1 comprenant en outre un revêtement (410) disposé sur au moins une partie d'une surface externe du segment intermédiaire (424).

3. Sonde d'électrochirurgie selon la revendication 2 dans laquelle le revêtement comprend un matériau isolant choisi dans le groupe constitué par le polydiméthylsiloxane (PDMS), le polyméthacrylate de méthyle (PMMA), le polycarbonate, le polytétrafluoroéthylène (PTFE), le polychlorure de vinyle (PVC), la polysulfone, le polystyrène, le polyméthylpentène, le polypropylène, le polyéthylène, le polyfluorure de vinylidène, l'ABS (copolymère acrylonitrile-butadiène-styrène), un polymère d'oléfine cyclique (COP), un copolymère d'oléfine cyclique (COC), et des combinaisons de ceux-ci.

4. Sonde d'électrochirurgie selon la revendication 1 dans laquelle l'ouverture dans le segment intermédiaire (424) est au moins aussi grande qu'une surface en section transversale du passage d'écoulement intérieur ; ou de manière facultative dans laquelle le segment de pointe (422) inclut l'un d'une pointe de lame et d'une pointe arrondie ; ou de manière facultative dans laquelle le segment de pointe (422) est formé dans le tube creux (423) ; ou de manière facultative dans laquelle le segment de pointe (422) est formé séparément du segment intermédiaire et est attaché au segment intermédiaire.

5. Sonde d'électrochirurgie selon la revendication 1 dans laquelle le tube est conducteur.

6. Sonde d'électrochirurgie selon la revendication 5 dans laquelle le tube inclut une série de découpes s'étendant à travers la paroi du tube, les découpes résultant en un tube flexible ; et de manière facultative dans laquelle la série de découpes forme un motif en spirale interrompue.

7. Système d'électrochirurgie, comprenant : une pièce à main d'électrochirurgie (140) comprenant un segment de corps allongé et un segment d'échappement, la pièce à main ayant un passage d'écoulement principal s'étendant longitudinalement à travers elle, la pièce à main ayant en outre un second passage s'étendant longitudinalement à travers une partie distale du segment de corps, le second passage se terminant dans un contact électrique, le segment d'échappement ayant un diamètre en section transversale supérieur à un diamètre en section transversale du passage d'écoulement principal ; et une sonde d'électrochirurgie selon la revendication 1, conçue pour être reliée de manière amovible à la pièce à main d'électrochirurgie (140) et
un connecteur (130) pouvant être couplé à la pièce à main d'électrochirurgie (140) et à la sonde d'électrochirurgie ;
dans lequel le segment de contact électrique et une partie du segment intermédiaire sont disposés de manière amovible à l'intérieur de la pièce à main d'électrochirurgie de telle sorte que le segment de contact électrique est positionné à l'intérieur du second passage et que l'ouverture du segment intermédiaire est positionnée à l'intérieur du segment d'échappement.

8. Système d'électrochirurgie selon la revendication 7 comprenant en outre un revêtement disposé sur au moins une partie d'une surface externe du segment intermédiaire (424).

9. Système d'électrochirurgie selon la revendication 7 dans lequel le segment d'échappement est un capuchon qui est formé séparément du segment de corps et attaché à une extrémité distale du segment de corps, et est construit en un matériau permettant de recevoir de manière amovible une sonde d'électrochirurgie.

10. Système d'électrochirurgie selon la revendication 7 dans lequel le segment d'échappement est une extension du segment de corps et est formé simultanément avec le segment de corps, du ou des mêmes matériaux que le segment de corps.

11. Système d'électrochirurgie selon la revendication 7 dans lequel le contact électrique est un réceptacle électrique concentrique.

12. Système d'électrochirurgie selon la revendication 7 dans lequel le segment de pointe de la sonde d'électrochirurgie (422) est formé dans le tube creux.
